# FASCICULE DE BREVET EUROPEEN

(11) **EP 1 024 767 B1**
(45) Date de publication et mention de la délivrance du brevet: **08.10.2003**
(21) Numéro de dépôt: 98950167.1
(22) Date de dépôt: 21.10.1998
(51) Int. Cl.: A61F 2/38

(54) **PROTHESE DU GENOU COMPORTANT UNE CALE TIBIALE D'EPAISSEUR**
KNIEPROTHESE MIT EINEM TIBIALEN VERDICKUNGSKEIL
KNEE PROSTHESIS COMPRISING A TIBIAL WEDGE

(30) Priorité: 21.10.1997 FR 9713162
(43) Date de publication de la demande: 09.08.2000
(73) Titulaire: Aesculap, 52000 Chaumont (FR)
(72) Inventeur: JERMANN, Jean-Louis, F-52000 Chaumont (FR); MIEHLKE, Rolf, K., D-48167 Munster (DE)
(74) Mandataire: Eidelsberg, Victor Albert
(86) Numéro de dépôt international: FR9802249
(87) Numéro de publication internationale: WO99020207

(56) Documents cités:
- EP-A- 0 538 987
- US-A- 5 387 241
- US-A- 5 458 637

## Description

La présente invention est relative aux prothèses du genou du type comportant notamment une partie tibiale, destinée à être fixée sur le tibia préparé et comprenant un plateau tibial destiné à coopérer par une première face avec au moins un élément méniscal et par une seconde face avec un plan de résection du tibia, une quille tibiale solidaire du plateau tibial et destinée à pénétrer dans le tibia pour la fixation de la partie tibiale sur celui-ci, et au moins une cale tibiale d'épaisseur destinée à être interposée à plat entre la seconde face du plateau tibial et le tibia pour compenser une absence osseuse locale entre le tibia et le plan de résection, cette cale tibiale coopérant par ailleurs avec ladite partie tibiale par l'intermédiaire de moyens complémentaires de fixation portés par la cale tibiale et ladite partie tibiale, les moyens complémentaires de fixation étant du type à saillie et logement. Une telle prothèse du genou est connue du document US-A-5 458 637.

Une telle cale tibiale de compensation est utilisée, par exemple, lorsqu'il existe une lacune ou absence d'os au-dessous du plan de résection tibiale et qu'une nouvelle résection ne peut pas être effectuée, comme cela se produit notamment en cas de reprise d'une prothèse du genou unicompartimentale ou en cas de mauvaise qualité osseuse, spécialement en cas d'arthrose.

L'invention a pour but de fournir un montage particulièrement simple et fiable de la cale tibiale sur la partie tibiale de la prothèse.

A cet effet, la prothèse selon l'invention est caractérisée en ce que les moyens complémentaires de fixation sont portés par la cale tibiale et la base de la quille tibiale.

Ainsi, dans le mode de montage selon l'invention, il suffit de faire pénétrer une saillie dans un logement pour assurer le montage et la retenue de la cale tibiale sur la base de la quille tibiale.

Suivant un mode de réalisation préféré, la saillie est portée par la base de la quille tibiale et le logement est ménagé dans la cale tibiale ; cette saillie peut constituer la base d'une nervure d'ancrage dans le tibia, solidaire de la quille tibiale.

La saillie présente avantageusement, à distance de son extrémité libre, un col rétréci destiné à recevoir au moins une excroissance de verrouillage prévue à l'entrée du logement.

De préférence, pour permettre une déformation de matière en vue d'un serrage élastique entre le logement et la saillie et/ou la pénétration de la saillie dans le logement, les moyens complémentaires de fixation comportent au moins une rainure ; cette rainure peut s'étendre soit le long du logement, sur une partie au moins de la longueur et à faible distance de celui-ci, pour définir avec lui une languette de fléchissement, soit dans la saillie, le long et sur une partie au moins de la longueur de celle-ci, jusqu'à son extrémité libre, pour définir deux languettes de fléchissement.

La saillie et le logement peuvent avoir une direction générale qui passe par l'axe de la quille tibiale.

Pour le montage d'une cale tibiale d'un côté ou de l'autre de la quille tibiale, cette dernière comporte une partie desdits moyens de fixation, par exemple deux saillies, symétriquement par rapport au plan médian antéro-postérieur de la quille tibiale.

Par exemple, les moyens complémentaires de fixation sont agencés pour que la cale tibiale s'engage par glissement sur la base de la quille tibiale par déplacement sensiblement perpendiculairement aux faces du plateau tibial et parallèlement à l'axe de la quille tibiale. En variante, les moyens complémentaires de fixation sont agencés pour que la cale tibiale s'engage sur la base de la quille tibiale par déplacement sensiblement parallèlement aux faces du plateau tibial et perpendiculairement à l'axe de la quille tibiale.

On comprendra bien l'invention à la lecture du complément de description qui va suivre et en référence au dessin annexé qui fait partie de la description et dans lequel :
Figs. 1 et 2 sont des vues de dessous montrant respectivement la cale tibiale et la partie tibiale d'une prothèse du genou établie suivant un mode de réalisation préféré de l'invention ;
Fig. 3 est une vue analogue aux Figs. 1 et 2 et montre la cale tibiale en place sous et contre le plateau tibial, ce dernier n'étant montré qu'en partie ;
Fig. 4 est une coupe verticale arrachée suivant la ligne IV-IV de la Fig. 3 ; et
Fig. 5 est une vue analogue à la Fig. 3, relativement à une variante de réalisation.

On a représenté sur le dessin la partie tibiale 1 d'une prothèse du genou établie suivant un mode de réalisation préféré de l'invention.

De manière connue, cette partie tibiale 1 comporte un plateau tibial 2 et une quille tibiale 3, solidaire du plateau et assemblée à celui-ci par tout moyen approprié ou d'une seule pièce avec lui.

Le plateau tibial 2 est destiné à coopérer, par sa face supérieure 4, avec au moins un élément méniscal prothétique (non représenté) et, par sa face inférieure 5, avec un plan de résection P du tibia (non représenté). La quille tibiale 3 est destinée à pénétrer dans le tibia pour la fixation de la partie tibiale 1 sur celui-ci.

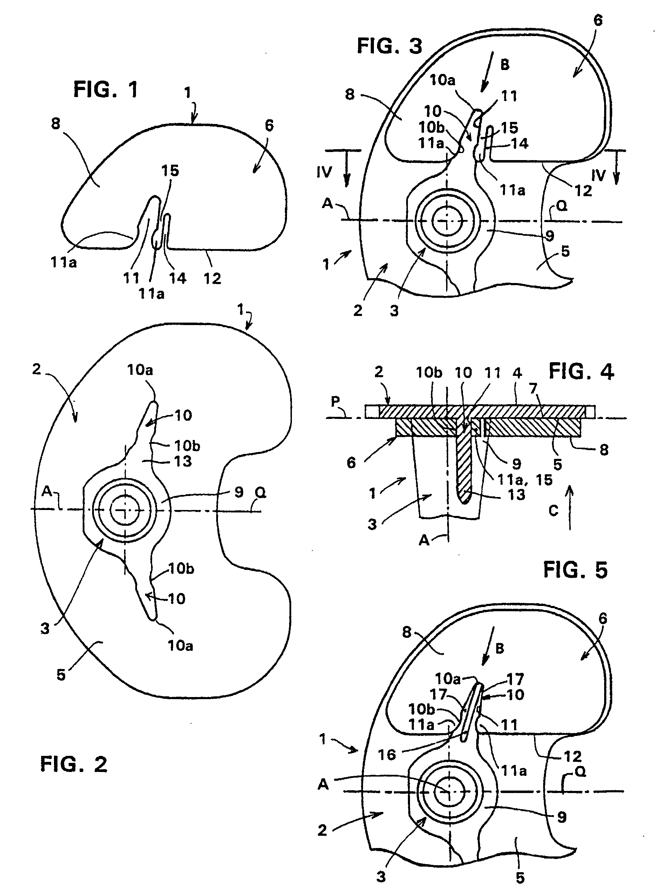

Comme indiqué précédemment, pour compenser une absence ou lacune osseuse sous le plan de résection P, représenté en traits mixtes sur la Fig. 4, dans les conditions et pour les raisons également indiquées précédemment, la prothèse comporte au moins une cale tibiale d'épaisseur 6 qui est destinée à être interposée à plat entre le plateau tibial 2 et le plan de résection P. Par sa face supérieure 7, la cale tibiale 6 est en appui contre la face inférieure 5 du plateau tibial 2 et, par sa face inférieure 8, elle est en appui contre le tibia, au-dessous du plan de résection P coïncidant avec la face inférieure 5 du plateau tibial. Comme montré sur les Figs. 3 et 5, le contour extérieur courbe de la cale 6 est légèrement en retrait du contour associé du plateau tibial, pour la position assemblée.

La cale tibiale 6 est montée sur la base 9 de la quille tibiale par l'intermédiaire de moyens complémentaires de fixation, qui sont portés par la cale tibiale 6 et par la base 9 de la quille 3 et qui comportent une saillie 10 pénétrant dans un logement 11.

Dans les modes de réalisation représentés, la saillie 10 est portée par la base 9 de la quille tibiale, et le logement 11 est ménagé dans la cale tibiale 6. Cet agencement est préféré pour des raisons d'encombrement, car le bord interne 12 de la cale 6 est relativement voisin de la quille 3 et pourrait difficilement comporter une saillie se logeant dans un évidement ménagé dans la quille, laquelle serait, dans ce cas, de résistance mécanique amoindrie.

Pour la retenue de la saillie 10 dans le logement 11, la saillie 10 présente, à distance de son extrémité libre 10a, un col rétréci 10b destiné à recevoir au moins une excroissance de verrouillage 11a prévue à l'entrée du logement 11. Dans les exemples représentés, il existe deux excroissances 11a, de part et d'autre du passage d'entrée dans le logement 11.

La saillie 10 et le logement 11 ont des formes allongées complémentaires, au jeu de serrage près. Ces formes allongées permettent un positionnement angulaire précis et correct de la cale sur la partie tibiale. Dans le sens radial, la position relative est définie par la butée de la saillie dans le logement. Dans les exemples représentés, la saillie 10 est effilée à partir du col 10b pour présenter une pointe arrondie à son extrémité libre 10a, et le logement 11 présente une forme complémentaire, à partir des excroissances 11a.

La quille tibiale 3 comporte deux saillies 10 qui sont situées symétriquement par rapport au plan médian antéro-postérieur Q passant par l'axe A de la quille (Figs. 2, 3 et 5). La présence de ces deux saillies 10 permet de placer une cale tibiale 6 sélectivement à gauche ou à droite.

Les saillies 10 constituent au surplus la base de raccordement avec le plateau tibial 2 de nervures d'ancrage 13 qui font partie intégrante de la quille 3 et qui sont destinées à pénétrer dans le tibia. Les nervures 13 sont en forme de sifflet, pour se fondre avec le corps de la quille 3 à distance du plateau tibial 2 (Fig. 4).

Comme montré sur les Figs. 2, 3 et 5, la direction générale des saillies 10 et du logement 11 de la cale 6 passe par l'axe A de la quille tibiale 3, la mise en coopération de la cale 6 avec la quille 3, par la saillie 10 de celle-ci, se faisant soit, comme montré par la flèche B (Figs. 3 et 5), par glissement suivant une direction transversale parallèle à la direction générale de la saillie 10 et du logement 11, sensiblement parallèlement aux faces 4 et 5 du plateau tibial 2 et perpendiculairement à l'axe A de la quille tibiale, soit, comme montré par la flèche C (Fig. 4), sensiblement perpendiculairement aux faces du plateau et parallèlement à l'axe de la quille tibiale.

Pour permettre un serrage élastique de la saillie 10 par le logement 11 et, dans le cas d'une mise en place transversale, le franchissement des excroissances d'entrée 11a du logement 11 par la saillie 10, il est nécessaire que la matière puisse se déformer.

Dans le mode de réalisation des Figs. 1 à 4, cette déformation de matière est rendue possible du fait que le logement 11 est bordé, au moins d'un côté, sur une partie au moins de sa longueur et à partir du bord 12 de la cale, par une rainure 14, d'une largeur appropriée, qui s'étend le long du logement 11 et à faible distance de celui-ci, pour définir avec lui une languette de fléchissement 15. Lors de la pénétration de la saillie 10 dans le logement 11, la languette 15 fléchit vers la rainure 14 pour l'écartement élastique des excroissances d'entrée 11a du logement 11. Dans le cas d'une mise en place transversale, la partie la plus large de la saillie 10, immédiatement avant le col 10b, franchit l'entrée du logement, après quoi la languette 15 revient par élasticité en position de verrouillage pour la coopération des excroissances 11a avec le col 10b.

La variante de la Fig. 5 ne diffère du mode de réalisation des Figs. 1 à 4 que par les moyens qui permettent la déformation de matière.

Dans ce mode de réalisation, c'est la saillie 10 qui présente, à partir de sa liaison avec la quille tibiale 3 et sur une hauteur appropriée, une rainure longitudinale 16, d'une largeur appropriée, qui s'étend, suivant la direction générale de la saillie 10, depuis l'extrémité libre 10a jusqu'au-delà du col 10b. La rainure centrale 16 définit donc, de chaque côté, une languette de fléchissement 17. Lors de la pénétration de la saillie 10 dans le logement 11, les languettes 17 fléchissent vers la rainure centrale 16.

Dans le cas d'une mise en place axiale de la cale 6, suivant la flèche C de la Fig. 4, il n'est pas nécessaire que le fléchissement des languettes 15 et 17 soit important, du fait qu'il s'agit seulement de faire serrer la saillie 10 par la paroi du logement. En revanche, dans le cas d'une mise en place transversale, suivant la flèche B des Figs. 3 et 5, le fléchissement doit être plus important car la partie la plus large de la saillie 10, avant le col 10b, doit franchir les excroissances 11a.

Bien entendu, l'invention n'est pas limitée aux modes de réalisation qui ont été décrits ; on pourrait au contraire concevoir diverses variantes sans sortir pour autant de son cadre, qui est défini par les revendications suivantes.

## Revendications

1. Prothèse du genou, comportant une partie tibiale (1) comprenant un plateau tibial (2) destiné à coopérer par une première face (4) avec au moins un élément méniscal et par une seconde face (5) avec un plan (P) de résection du tibia, une quille tibiale (3) solidaire du plateau tibial (2) et destinée à pénétrer dans le tibia pour la fixation de la partie tibiale (1) dans celui-ci, et au moins une cale tibiale d'épaisseur (6) destinée à être interposée à plat entre la seconde face (5) du plateau tibial (2) et le tibia pour compenser une absence osseuse locale entre le tibia et le plan de résection (P), cette cale tibiale (6) coopérant par ailleurs avec ladite partie tibiale (1) par l'intermédiaire de moyens complémentaires de fixation (10, 11) portés par la cale tibiale (6) et ladite partie tibiale, les moyens complémentaires de fixation étant du type à saillie (10) et logement (11), **caractérisée en ce que** les moyens complémentaires de fixation sont portés par la cale tibiale (6) et la base (9) de la quille tibiale (3).

2. Prothèse du genou selon la revendication 1, **caractérisée en ce que** la saillie (10) est portée par la base (9) de la quille tibiale (3) et le logement (11) est ménagé dans la cale tibiale (6).

3. Prothèse du genou selon la revendication 2, **caractérisée en ce que** la saillie (10) constitue la base d'une nervure (13) d'ancrage dans le tibia, solidaire de la quille tibiale (3).

4. Prothèse du genou selon l'une des revendications 1 à 3, **caractérisée en ce que** la saillie (10) présente, à distance de son extrémité libre (10a), un col rétréci (10b) destiné à recevoir au moins une excroissance de verrouillage (11a) prévue à l'entrée du logement (11).

5. Prothèse du genou selon l'une des revendications 1 à 4, **caractérisée en ce que** les moyens de fixation comportent au moins une rainure (14, 16) permettant une déformation de matière pour un serrage élastique entre le logement (11) et la saillie (10) et/ou la pénétration de la saillie dans le logement.

6. Prothèse du genou selon la revendication 5, **caractérisée en ce qu'**une rainure (14) s'étend, au moins d'un côté, le long du logement (11), sur une partie au moins de sa longueur et à faible distance de celui-ci, pour définir avec lui une languette de fléchissement (15).

7. Prothèse du genou selon la revendication 5, **caractérisée en ce que** la rainure (16) s'étend dans la saillie (10), le long et sur une partie au moins de la longueur de celle-ci, jusqu'à son extrémité libre (10a), pour définir deux languettes de fléchissement (17).

8. Prothèse du genou selon l'une des revendications 1 à 7, **caractérisée en ce que** la saillie (10) et le logement (11) ont une direction générale qui passe par l'axe (A) de la quille tibiale (3).

9. Prothèse du genou selon l'une des revendications 1 à 8, **caractérisée en ce que** la quille tibiale (3) comporte une partie desdits moyens de fixation symétriquement par rapport au plan médian antéro-postérieur (Q) de la quille tibiale (3).

10. Prothèse du genou selon l'une des revendications 1 à 9, **caractérisée en ce que** les moyens complémentaires de fixation sont agencés pour que la cale tibiale (6) s'engage par glissement sur la base (9) de la quille tibiale (3) par déplacement (C) sensiblement perpendiculairement aux faces (4, 5) du plateau tibial (2) et parallèlement à l'axe (A) de la quille tibiale (3).

11. Prothèse du genou selon l'une des revendications 1 à 9, **caractérisée en ce que** les moyens complémentaires de fixation sont agencés pour que la cale tibiale (6) s'engage sur la base (9) de la quille tibiale (3) par déplacement (B) sensiblement parallèlement aux faces (4, 5) du plateau tibial (2) et perpendiculairement à l'axe (A) de la quille tibiale (3).

## Patentansprüche

1. Knieprothese mit einem Schienbeinteil (1), das eine Schienbeinplatte (2), die dazu bestimmt ist, über eine erste Fläche (4) mit mindestens einem Meniskuselement und über eine zweite Fläche (5) mit einer Resektionsebene (P) des Schienbeins zusammenzuwirken, einen fest mit der Schienbeinplatte (2) verbundenen Schienbeinkegel (3), der dazu bestimmt ist, in das Schienbein einzudringen, um das Schienbeinteil (1) darin zu befestigen, und mindestens eine Schienbein-Unterlegplatte (6) aufweist, die dazu bestimmt ist, flach zwischen die zweite Fläche (5) der Schienbeinplatte (2) und das Schienbein eingeschoben zu werden, um ein lokales Fehlen von Knochen zwischen dem Schienbein und der Resektionsebene (P) zu kompensieren, wobei diese Schienbein-Unterlegplatte (6) außerdem mit dem Schienbeinteil (1) über komplementäre Befestigungsmittel (10, 11) zusammenwirkt, die von der Schienbein-Unterlegplatte (6) und dem Schienbeinteil getragen werden, wobei die komplementären Befestigungsmittel von der Art mit Vorsprung (10) und Sitz (11) sind, **dadurch gekennzeichnet, dass** die komplementären Befestigungsmittel von der Schienbein-Unterlegplatte (6) und der Basis (9) des Schienbeinkegels (3) getragen werden.

2. Knieprothese nach Anspruch 1, **dadurch gekennzeichnet, dass** der Vorsprung (10) von der Basis (9) des Schienbeinkegels (3) getragen wird und der Sitz (11) in der Schienbein-Unterlegplatte (6) ausgebildet ist.

3. Knieprothese nach Anspruch 2, **dadurch gekennzeichnet, dass** der Vorsprung (10) die Basis einer Rippe (13) zur Verankerung im Schienbein bildet, die fest mit dem Schienbeinkegel (3) verbunden ist.

4. Knieprothese nach einem der Ansprüche 1 bis 3, **dadurch gekennzeichnet, dass** der Vorsprung (10) in Abstand zu seinem freien Ende (10a) einen verengten Hals (10b) aufweist, der dazu bestimmt ist, mindestens eine Verriegelungsausstülpung (11a) aufzunehmen, die am Eingang des Sitzes (11) vorgesehen ist.

5. Knieprothese nach einem der Ansprüche 1 bis 4, **dadurch gekennzeichnet, dass** die Befestigungsmittel mindestens eine Nut (14, 16) aufweisen, die eine Materialverformung für eine elastische Klemmung zwischen dem Sitz (11) und dem Vorsprung (10) und/oder das Eindringen des Vorsprungs in den Sitz ermöglicht.

6. Knieprothese nach Anspruch 5, **dadurch gekennzeichnet, dass** eine Nut (14) sich zumindest auf einer Seite entlang des Sitzes (11) über zumindest einen Teil seiner Länge und in geringem Abstand zu diesem erstreckt, um mit ihm eine Biegezunge (15) zu definieren.

7. Knieprothese nach Anspruch 5, **dadurch gekennzeichnet, dass** die Nut (16) sich im Vorsprung (10) entlang diesem und zumindest über einen Teil von dessen Länge bis zu seinem freien Ende (10a) erstreckt, um zwei Biegezungen (17) zu definieren.

8. Knieprothese nach einem der Ansprüche 1 bis 7, **dadurch gekennzeichnet, dass** der Vorsprung (10) und der Sitz (11) eine allgemeine Richtung aufweisen, die durch die Achse (A) des Schienbeinkegels (3) verläuft.

9. Knieprothese nach einem der Ansprüche 1 bis 8, **dadurch gekennzeichnet, dass** der Schienbeinkegel (3) einen Teil der Befestigungsmittel symmetrisch in Bezug auf die von vorne nach hinten verlaufende Mittelebene (Q) des Schienbeinkegels (3) aufweist.

10. Knieprothese nach einem der Ansprüche 1 bis 9, **dadurch gekennzeichnet, dass** die komplementären Befestigungsmittel so gestaltet sind, dass die Schienbein-Unterlegplatte (6) durch eine Verschiebung (C) im wesentlichen senkrecht zu den Flächen (4, 5) der Schienbeinplatte (2) und parallel zur Achse (A) des Schienbeinkegels (3) durch Gleiten auf der Basis (9) des Schienbeinkegels (3) in Eingriff gelangt.

11. Knieprothese nach einem der Ansprüche 1 bis 9, **dadurch gekennzeichnet, dass** die komplementären Befestigungsmittel so gestaltet sind, dass die Schienbein-Unterlegplatte (6) durch eine Verschiebung (B) im wesentlichen parallel zu den Flächen (4, 5) der Schienbeinplatte (2) und senkrecht zur Achse (A) des Schienbeinkegels (3) auf der Basis (9) des Schienbeinkegels (3) in Eingriff gelangt.

## Claims

1. A knee prosthetic including a tibia part (1), which includes a tibia plate (2) that co-operates with at least one meniscal element by a first side (4), and co-operates with a tibia resection plan (P) by a second side (5), including:
a tibia pin (3) connected with the tibia plate (2) which penetrates into the tibia to fix the tibia part (1) into it; and
at least one compensating tibia block (6) which fits flatly between the second side (5) of the tibia plate (2) and the tibia itself, in order to compensate for a lack or absence of local bone tissue between the tibia and the resection plan (P), wherein said tibia block (6) also co-operates with said tibia part (1) by way of complementary fixating means (10, 11) that are implemented on the tibia block (6) and the tibia part, wherein the complementary fixating means are of the pin (10) and hole (11) type , **characterized in that** the complementary fixating means are implemented on the tibia block (6) and the base (9) of the tibia pin (3).

2. A knee prosthetic according to claim **1**, **characterized in that** the pin (10) is implemented on the base (9) of the tibia pin (3) and the hole (11) is implemented in the tibia block (6).

3. A knee prosthetic according to claim **2**, **characterized in that** the pin (10) constitutes the base of a ridge (13) anchoring within the tibia and which is connected with the tibia pin (3).

4. A knee prosthetic according to one of claims **1 to 3**, **characterized in that** the pin (10) features a shrunken shaft (10b) some distance away from its free extremity (10a), which will receive at least one locking bulge (11a) implemented at the entrance of the hole (11).

5. A knee prosthetic according to one of claims **1 to 4**,
**characterized in that** said fixating means include at least one ridge (14, 16) which allows for the alteration of the shape of the material to achieve an elastic clasping between the hole (11) and the pin (10), and/or the penetration of the pin in the hole.

6. A knee prosthetic according to claim **5**, **characterized in that** a ridge (14) extends along the hole (11) on at least one side, and on at least part of its length and a small distance away from it, in order to define a flexing plate (15).

7. A knee prosthetic according to claim **5**, **characterized in that** the ridge (16) extends in the pin (10), along and on at least part of its length until its free extremity (10a) in order to define two flexing plates (17).

8. A knee prosthetic according to one of claims **1 to 8**, **characterized in that** the pin (10) and the hole (11) have a general direction which intersects the axis A of the tibia pin (3).

9. A knee prosthetic according to any one of claims **1 to 8**, **characterized in that** the tibia pin (3) includes part of said fixating means, symmetrically compared to the median pre-posterior plan (Q) of the tibia pin (3).

10. A knee prosthetic according to one of claims **1 to 9**, **characterized in that** the complementary fixating means are implemented so that the tibia block (6) slides onto the base (9) of the tibia pin (3), by a movement (C) perpendicular to the sides (4, 5)of the tibia plate (2) and parallel to the axis (A) of the tibia pin (3).

11. A knee prosthetic according to one of claims **1 to 9**, **characterized in that** the complementary fixating means are implemented so that the tibia block (6) co operate with the base (9) of the tibia pin (3), by a movement (B) parallel to the sides (4, 5)of the tibia plate (2) and perpendicular to the axis (A) of the tibia pin (3).
